(19) Europäisches Patentamt
European Patent Office
Office européen des brevets

(11) Publication number : 0 349 087 B1

(12) EUROPEAN PATENT SPECIFICATION

(45) Date of publication of patent specification : 09.09.92 Bulletin 92/37

(51) Int. Cl.$^5$ : C07C 29/136, C07C 29/14

(21) Application number : 89201714.6

(22) Date of filing : 27.06.89

# (54) Process for the reduction of carbonyl compounds.

(30) Priority : 28.06.88 GB 8815328

(43) Date of publication of application : 03.01.90 Bulletin 90/01

(45) Publication of the grant of the patent : 09.09.92 Bulletin 92/37

(84) Designated Contracting States : BE CH DE FR GB IT LI NL

(56) References cited :
GB-A- 1 529 619
US-A- 4 072 720
US-A- 4 418 227

(56) References cited :
JOURNAL OF THE CHEMICAL SOCIETY, CHEMICAL COMMUNICA- TIONS, ROYAL SOCIETY OF CHEMISTRY, London, Index 1986 PIET W.N.M. VANLEEUWEN et al. "Platinom Hydroformyla- tion Catalysts containing Diphenylphosphine Oxide Ligands" pages 31-33

(73) Proprietor : SHELL INTERNATIONALE RESEARCH MAATSCHAPPIJ B.V.
Carel van Bylandtlaan 30
NL-2596 HR Den Haag (NL)

(72) Inventor : Van Leeuwen, Petrus Wilhelmus Nicolaas Maria
Badhuisweg 3
NL-1031 CM Amsterdam (NL)
Inventor : Roobeek, Cornelis Franciscus
Badhuisweg 3
NL-1031 CM Amsterdam (NL)

EP 0 349 087 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for the preparation of primary or secondary alcohols by the reduction of aldehydes or ketones. In particular it relates to a hydrogenation process in which a homogeneous catalyst system comprising a platinum compound, a phosphorus compound, and an acid is used.

P.W.N.M. van Leeuwen et al, J. Chem. Soc., Chem. Commun., 1986, 31 to 33, describes the hydroformylation of certain alkenes using a catalyst system comprising a platinum compound and a phosphorus compound of formula $Ph_2POH$. The products of the hydroformylation are mixtures of aldehydes or ketones and alcohols. Presumably the alcohols are formed by the hydrogenation of carbonyl compounds.

Surprisingly, it has now been found that aldehydes and ketones can advantageously be hydrogenated using a platinum compound, a phosphorus compound and an acid.

Accordingly, the present invention provides a process for the preparation of a primary or secondary alcohol, which comprises hydrogenating respectively an aldehyde or ketone in the presence of a catalyst system comprising a platinum compound, an acid having a pKa in the range of from 2 to 10.5 and a phosphorus compound of general formula

$$R^1R^2POH \qquad (I)$$

in which $R^1$ and $R^2$ independently represent an alkyl group or an optionally substituted phenyl group, at a temperature in the range of from 20 to 150°C and a pressure in the range of from 1 to 100 bar.

The process according to the invention affords the alcohol products at a very high rate, which is very surprising.

Preferably the aldehyde or ketone is a compound of general formula

$$R^3CHO \qquad (II) \qquad \text{or } R^3COR^4 \qquad (III)$$

in which each of $R^3$ and $R^4$ is independently selected from optionally substituted alkyl, alkenyl, aryl, cycloalkyl and cycloalkenyl, or $R^3$ and $R^4$ together form an optionally substituted alkylene chain optionally containing one or more carbon-carbon double bonds.

In this specification, unless otherwise stated, any alkyl or alkenyl group preferably has from 1 to 10, more preferably from 3 to 7 carbon atoms. Any aryl group is preferably a phenyl group. Any cycloalkyl or cycloalkenyl group preferably has from 5 to 7 carbon atoms. An alkylene chain optionally containing one or more carbon-carbon double bonds preferably has from 4 to 6 carbon atoms. Optional substituents, when present, are preferably selected from alkyl, alkoxy, halogen, alkoxyalkyl and alkoxycarbonyl groups.

Very surprisingly, it has been found that when compounds containing a non-conjugated carbon-carbon double bond are hydrogenated according to the process of the invention, the carbon-carbon double bond is remarkably resistant to reduction. Accordingly, the process according to the invention is particularly advantageous for the selective reduction of aldehydes and ketones containing a non-conjugated carbon-carbon double bond.

It is particularly surprising that aldehydes may successfully be reduced by the process according to the invention. Thus ruthenium and rhodium based catalysts can be poisoned by carbon monoxide which may be liberated during hydrogenation.

The catalyst system used in the process according to the invention may be generated either by mixing each of the individual components, or by mixing substances which do not correspond to each of the individual components, but which in combination generate the catalyst system.

The platinum compound may conveniently be a salt of platinum, for example a platinum carboxylate such as acetate, or a complex of platinum, for example platinum bis(cyclo-octa-1,5-diene) or platinum tris(ethylene).

The acid having a pKa in the range of from 2 to 10.5 may be an inorganic acid such as phosphoric acid or an organic acid such as a carboxylic acid or phenol. Preferably the acid is a carboxylic acid.

When the acid having a pKa in the range of from 2 to 10.5 is a carboxylic acid, it may be an aliphatic carboxylic acid such as an alkanoic acid, e.g., ethanoic acid, propanoic acid, butanoic acid or 2-methylpropanoic acid; or an aromatic carboxylic acid such as benzoic acid.

The phosphorus compound of general formula (I) is preferably diphenylphosphinous acid.

Preferably the process according to the invention is effected at a temperature in the range of from 80 to 120°C.

The pressure is preferably in the range of from 20 to 60 bar.

The molar ratio of phosphorus compound of formula (I) to platinum compound is conveniently in the range of from 1:1 to 4:1, but could be higher. Preferably the ratio is in the range of from 2:1 to 4:1.

In generating the catalyst system, the platinum compound and the phosphorus compound of general formula (I) may be introduced separately or in the form of a single complex. For example they may be introduced in the form of a complex of general formula

$$(R^1R^2PO)(R^1R^2POH)_2PtH \qquad (IV)$$

in which $R^1$ and $R^2$ are as defined for the general formula (I). In this case the molar ratio of phosphorus compound to platinum compound is, of course, exactly 3:1.

The molar ratio of acid having a pKa in the range of from 2 to 10.5 to platinum compound may conveniently be in the range of from 1:1 to 100:1.

It will be appreciated that aldehydes tend to react with air to afford carboxylic acids. Consequently impure samples of aldehydes often contain carboxylic acids. When such impure samples of aldehyde are used in the process according to the invention, it may be unnecessary to use any additional acid.

The process is optionally effected in the presence of a solvent. Suitable solvents include aromatic hydrocarbons such as toluene, haloalkanes such as dichloromethane, ethers such as dioxan or tetrahydrofuran, esters such as ethyl ethanoate, and alcohols such as ethanol.

In certain cases it is advantageous to use an additional pi-acceptor ligand in the catalyst system. The additional pi-acceptor ligand may be, for example, carbon monoxide, ethene, triphenylphosphine, tri(p-tolyl)phosphine or tricyclohexylphosphine. Additional gaseous pi-accetor ligands may conveniently be present at a pressure in the range of from 5 to 10 bar. Phosphines may conveniently be present at a molar ratio of phosphine to platinum of below 2.

The alcohol products of the process according to the invention may be recovered using techniques well known in the art of homogeneous catalysis, such as distillation under reduced pressure. Advantageously platinum may be recovered from the reaction product by chromatography over phosphine-carrying resins, and the phosphine oxides may be removed by percolation over alumina.

The following examples illustrate the invention.

## Example 1

### Hydrogenation of 3-(4-Methyl-3-cyclohexen-1-yl)-1-butanal

A 100ml autoclave (Hastelloy C) was charged with 20ml of toluene, 15 mmol of 3-(4-methyl-3-cyclohexen-1-yl)butanal (prepared by the hydroformylation of limonene, as described in EP-A-54986), 0.05 mmol of $(Ph_2PO)(Ph_2POH)_2PtH$ and 0.5 mmol of $CH_3COOH$. The autoclave was then pressurized with 40 bar $H_2$ and then heated to 95°C. The reaction mixture was kept at this temperature for 0.5 hours. The mixture was then allowed to cool and was analysed thereafter using gas-liquid chromatography. The conversion of 3-(4-methyl-3-cyclohexen-1-yl)butanal was 37.5%, with a selectivity to 3-(4-Methyl-3-cyclohexen-1-yl)-1-butanol of 90%, i.e. less than 10% of the olefinic double bond had been hydrogenated. From the conversion, amount of catalyst and the reaction time an average turnover per hour is calculated of 200 mol of product per mol of catalyst per hour.

## Examples 2 to 11

The method of Example 1 was repeated using different aldehydes and ketones. The amounts of substances used and the rates of reaction are given in Table 1. The rate is the average rate in mol product per mol of catalyst per hour measured over 30-50% conversion.

Table 1

| Example Number | $(Ph_2POH)_3Pt$ (mmol) | Acid (mmol) | Additional ligand(s) (bar) | Substrate (mmol) | Solvent (ml) | Product | Rate $m.m^{-1}.h^{-1}$ |
|---|---|---|---|---|---|---|---|
| 2 * | 0.02 | $(CH_3)_2CHCOOH$ (4) | none | $(CH_3)_2CHCHO$ (110) | ethanol (20) | i-butanol $(CH_3)_2CHCH_2OH$ | 1000 |
| 3 * | 0.02 | $(CH_3)_2CHCOOH$ (4) | $C_2H_4$ (5) | $(CH_3)_2CHCHO$ (110) | ethanol (20) | $(CH_3)_2CHCH_2OH$ | 2500 |
| 4 | 0.02 | $(CH_3)_2CHCOOH$ (4) | $C_2H_4$ (5) | $(CH_3)_2CHCHO$ (110) | toluene (20) | $(CH_3)_2CHCH_2OH$ | 4500 |
| 5 * | 0.01 | $(CH_3)_2CHCOOH$ (9) | none | $(CH_3)_2CHCHO$ (220) | none | $(CH_3)_2CHCH_2OH$ | 9000 |

**Table 1 (Continued)**

| Example Number | $(Ph_2POH)_3Pt$ (mmol) | Carboxylic acid (mmol) | Additional ligand(s) (bar) | Substrate (mmol) | Solvent (ml) | Product | Rate $m.m^{-1}.h^{-1}$ |
|---|---|---|---|---|---|---|---|
| 6 | 0.02 | $CH_3COOH$ (0.1) | none | $(CH_3)_2CHCHO$ (22) | toluene (20) | $(CH_3)_2CHCH_2OH$ | 1500 |
| Comparison | 0.02 | none | none | $CH_3)_2CHCHO$ (22) | toluene (20) | $(CH_3)_2CH_2CH_2OH$ | 200 |
| 7 | 0.02 | $CH_3COOH$ (10) | none | $CH_3COCH_3$ (275) | none | $(CH_3)_2CHOH$ | 500 |
| Comparison | 0.01 | none | none | $CH_3COCH_3$ (275) | none | $(CH_3)_2CHOH$ | <10 |
| 8 | 0.02 | $(CH_3)_2CHCOOH$ (4) | none | cyclohexanone (200) | none | cyclohexanol | 1200 |

Table 1 (Continued)

| Example Number | $(Ph_2POH)_3Pt$ (mmol) | Carboxylic acid (mmol) | Additional ligand(s) (bar) | Substrate (mmol) | Solvent (ml) | Product | Rate $m.m^{-1}.h^{-1}$ |
|---|---|---|---|---|---|---|---|
| 9 | 0.01 | $(CH_3)_2CHCOOH$ (5) | none | $(CH_3)_2CHCHO$ (220) | none | $(CH_3)_2CHCH_2OH$ | 2800 |
| 10 | 0.01 | $(CH_3)_2CHCOOH$ (5) | CO (5) $H_2$ (35) | $(CH_3)_2CHCHO$ (220) | none | $(CH_3)_2CHCH_2OH$ | 1100 |
| 11 Comparison | 0.02 | 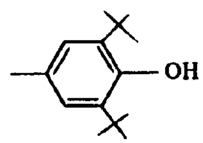 (0.1) | none | $(CH_3)_2CHCHO$ | toluene (20) | $(CH_3)_2CHOH$ | 220 |

* Undistilled substrate; contained 4% $(CH_3)_2CHCOOH$.

Example 12

The method of Example 1 was repeated using 20.0 mmol of 5-formyl-bicyclo[2,2,1]-2-heptene (exo/endo, prepared by the Diels Alder addition of acroleine to cyclopentadiene) as the aldehyde, 0.05 mmol of $(Ph_2PO)(Ph_2POH)_2PtH$ and 1 mmol of $PPh_3$ as additional ligand. The reaction temperature was 110°C. The pressure applied was 38 bar of $H_2$ and 2 bar of CO. After 1 hour of reaction time 50% was converted to 5-hydroxymethyl-bicyclo- [2,2,1]-2-heptene with a selectivity of 98%. The calculated rate is 200 mol per mol per hour.

Examples 13 to 18

These Examples show the effect of varying the presence and/or using additional pi-acceptor ligands on the hydrogenation of 5-formyl-bicyclo[2,2,1]-2-heptene. The amounts of reactants and solvent and the temperature were the same as in Example 12.

Table 2

| Example | $PPh_3$/ Pt ratio | bar $H_2$ | bar CO | bar $C_2H_4$ | conv % | sel % | rate |
|---|---|---|---|---|---|---|---|
| 13 | | 40.00 | | | 90.00 | 50.00 | 400.00 |
| 14 | 1.00 | 20.00 | 20.00 | 10.00 | 10.00 | 98.00 | 40.00 |
| 15 | 2.00 | 40.00 | | | 25.00 | 65.00 | 100.00 |
| 16 | 1.00 | 40.00 | | | 50.00 | 60.00 | 200.00 |
| 17 | 1.00 | 20.00 | | | 45.00 | 65.00 | 180.00 |
| 18 | 1.00 | 38.00 | 2.00 | | 50.00 | 98.00 | 200.00 |

Example 19

10-decenal (5ml) was hydrogenated using 0.05 mmol of $(Ph_2PO)(Ph_2POH)_2PtH$, 25ml of toluene, and 0.5 mmol of isobutyric acid at a pressure of 40 bar $H_2$ and a temperature of 95°C. After 55 minutes the conversion was 56%, with a selectivity of 59% to the unsaturated aldehyde according to gas chromatographic analysis.

Example 20

The method of Example 19 was repeated but with $(PPh_2OH)(PPh_2O)PtH(P(p\text{-}tolyl_3))$as the platinum compound. The conversion was 63% after 70 minutes with a selectivity of 52%.

Example 21

The method of Example 20 was repeated but using a platinum compound of formula $(PPh_2OH)(PPh_2O)PtH(P\text{-}(cyclohexyl)_3)$. After 60 minutes a conversion was obtained of 81% with a selectivity of 59%.

Example 22

The method of Example 21 was repeated except that carbon monoxide was additionally supplied at a pressure of 2 bar. The selectivity increased to 98%, at 120°C, and at 17% conversion after one hour.

Example 23 to 25

Hydrogenation of 6-methyl-5-hepten-2-one was carried out with the catalysts and conditions of Examples 19 to 22.

| Example | time minutes | conversion % | selectivity % |
|---|---|---|---|
| 23 | 100 | 17 | 59 |
| 24 | 150 | 9 | 61 |
| 25 | 140 | 14 | 61 |

**Claims**

1. A process for the preparation of a primary or secondary alcohol, which comprises hydrogenating respectively an aldehyde or ketone in the presence of a catalyst system comprising a platinum compound, an acid having a pKa in the range of from 2 to 10.5 and a phosphorus compound of general formula

$$R^1R^2POH \qquad (I)$$

in which $R^1$ and $R^2$ independently represent an alkyl group or an optionally substituted phenyl group, at a temperature in the range of from 20 to 150°C and a pressure in the range of from 1 to 100 bar.

2. A process as claimed in claim 1, in which the aldehyde or ketone is a compound of general formula

$R^3CHO$ (II) or $R^3COR^4$ (IV)

in which each of $R^3$ and $R^4$ is independently selected from optionally substituted alkyl, alkenyl, aryl, cycloalkyl and cycloalkenyl, or $R^3$ and $R^4$ together form an optionally substituted alkylene chain optionally containing one or more carbon-carbon double bonds.

3. A process as claimed in claim 1 or claim 2, in which the aldehyde or ketone contains a non-conjugated carbon-carbon double bond.

4. A process as claimed in any one of claims 1 to 3, in which an aldehyde is used.

5. A process as claimed in any one of claims 1 to 4, in which the platinum compound is platinum bis(cycloocta-1,5-diene), platinum acetate or platinum tris(ethylene).

6. A process as claimed in any one of claims 1 to 5, in which the acid having a pKa in the range of from 2 to 10.5 is a carboxylic acid.

7. A process as claimed in claim 6, in which the carboxylic acid is selected from ethanoic acid, propanoic acid, 2-methylpropanoic acid and butanoic acid.

8. A process as claimed in any one of claims 1 to 7, in which the phosphorus compound is diphenylphosphinous acid.

9. A process as claimed in any one of claims 1 to 8, in which the temperature is in the range of from 80 to 120°C.

10. A process as claimed in any one of claims 1 to 9, in which the pressure is in the range of from 20 to 60 bar.

## Patentansprüche

1. Verfahren zur Herstellung eines primären oder sekundären Alkohols, umfassend das Hydrieren eines Aldehyds bzw. Ketons in Gegenwart eines Katalysatorsystems, umfassend eine Platinverbindung und eine Säure mit einem pKa-Wert im Bereich von 2 bis 10,5 und eine Phosphorverbindung der allgemeinen Formel

$R^1R^2POH$ (I)

in der $R^1$ und $R^2$ unabhängig voneinander eine Alkylgruppe oder eine gegebenenfalls substituierte Phenylgruppe bedeuten, bei einer Temperatur im Bereich von 20 bis 150°C und einem Druck im Bereich von 1 bis 100 bar.

2. Verfahren nach Anspruch 1, wobei der Aldehyd oder das Keton eine Verbindung der allgemeinen Formel

$R^3CHO$ (II) oder $R^3COR^4$ (IV)

ist, in der jeder Rest $R^3$ und $R^4$ unabhängig ausgewählt ist aus gegebenenfalls substituiertem Alkyl, Alkenyl, Aryl, Cycloalkyl und Cycloalkenyl, oder $R^3$ und $R^4$ zusammen eine gegebenenfalls substituierte Alkylenkette bedeuten, die gegebenenfalls eine oder mehrere Kohlenstoff-Kohlenstoff-Doppelbindungen enthält.

3. Verfahren nach Anspruch 1 oder 2, wobei der Aldehyd oder das Keton eine nicht-konjugierte Kohlenstoff-Kohlenstoff-Doppelbindung enthält.

4. Verfahren nach einem der Ansprüche 1 bis 3, wobei ein Aldehyd verwendet wird.

5. Verfahren nach einem der Ansprüche 1 bis 4, wobei die Platinverbindung Platin-bis(cyclo-octa-1,5-dien), Platinacetat oder Platin-tris(ethylen) ist.

6. Verfahren nach einem der Ansprüche 1 bis 5, wobei die Säure mit einem pKa-Wert im Bereich von 2 bis 10,5 eine Carbonsäure ist.

7. Verfahren nach Anspruch 6, wobei die Carbonsäure ausgewählt ist aus Ethansäure (Essigsäure), Propansäure, 2-Methylpropansäure und Butansäure (Buttersäure).

8. Verfahren nach einem der Ansprüche 1 bis 7, wobei die Phosphorverbindung Diphenylphosphinsäure ist.

9. Verfahren nach einem der Ansprüche 1 bis 8, wobei die Temperatur im Bereich von 80 bis 120°C liegt.

10. Verfahren nach einem der Ansprüche 1 bis 9, wobei der Druck im Bereich von 20 bis 60 bar liegt.

## Revendications

1. Un procédé pour la préparation d'un alcool primaire ou secondaire, qui comprend l'hydrogénation d'un aldéhyde ou d'une cétone, respectivement, en présence d'un système catalytique comprenant un composé du platine, un acide ayant un pKa compris entre 2 et 10,5 et un composé du phosphore de la formule générale

$$R^1R^2POH \qquad (I)$$

où $R^1$ et $R^2$ indépendamment représentent un groupe alcoyle ou un groupe phényle éventuellement substitué, à une température comprise entre 20 et 150°C et une pression comprise entre 1 et 100 bars.

2. Un procédé selon la revendication 1, dans lequel l'aldéhyde ou la cétone est un composé de formule générale

$$R^3CHO \qquad (II) \qquad \text{ou } R^3COR^4 \qquad (III)$$

où chacun de $R^3$ et $R^4$ est choisi indépendamment parmi des groupes alcoyle, alcényle, aryle, cycloalcoyle et cycloalcényle éventuellement substitués, ou $R^3$ et $R^4$ forment ensemble une chaîne alcoylène éventuellement substituée contenant une ou plusieurs doubles liaisons carbone-carbone.

3. Un procédé selon la revendication 1 ou la revendication 2, dans lequel l'aldéhyde ou la cétone contient une double liaison carbone-carbone non-conjuguée.

4. Un procédé selon l'une quelconque des revendications 1 à 3, dans lequel on utilise un aldéhyde.

5. Un procédé selon l'une quelconque des revendications 1 à 4, dans lequel le composé du platine est du platine bis(cyclo-octa-1,5-diène), de l'acétate de platine ou du platine tris(éthylène).

6. Un procédé selon l'une quelconque des revendications 1 à 5, dans lequel l'acide ayant un pKa compris entre 2 et 10,5 est un acide carboxylique.

7. Un procédé selon la revendication 6, dans lequel l'acide carboxylique est choisi parmi l'acide éthanoïque, l'acide propanoïque, l'acide 2-méthylpropanoïque et l'acide butanoïque.

8. Un procédé selon l'une quelconque des revendications 1 à 7, dans lequel le composé du phosphore est de l'acide diphénylphosphineux.

9. Un procédé selon l'une quelconque des revendications 1 à 8, dans lequel la température est comprise entre 80 et 120°C.

10. Un procédé selon l'une quelconque des revendications 1 à 9, dans lequel la pression est comprise entre 20 et 60 bars.